# Europäisches Patentamt

# European Patent Office

# Office européen des brevets

(19)

(11) Publication number : **0 401 377 B1**

(12)
# EUROPEAN PATENT SPECIFICATION

(45) Date of publication of patent specification :
**15.09.93 Bulletin 93/37**

(51) Int. Cl.⁵ : **A61K 49/00**

(21) Application number : **89910937.5**

(22) Date of filing : **03.10.89**

(86) International application number :
**PCT/JP89/01009**

(87) International publication number :
**WO 90/03800 19.04.90 Gazette 90/09**

(54) **IRON-CONTAINING PREPARATION FOR USE AS NMR CONTRAST MEDIUM.**

(30) Priority : **04.10.88 JP 250664/88**
**27.09.89 JP 252895/89**

(43) Date of publication of application :
**12.12.90 Bulletin 90/50**

(45) Publication of the grant of the patent :
**15.09.93 Bulletin 93/37**

(84) Designated Contracting States :
**DE FR IT NL SE**

(56) References cited :
**EP-A- 0 186 616**
**JP-A- 6 016 936**
**US-A- 4 615 879**
**US-A- 4 675 173**

(73) Proprietor : **OTSUKA PHARMACEUTICAL CO., LTD.**
**9, Kandatsukasacho 2-chome**
**Chiyoda-ku Tokyo 101 (JP)**

(72) Inventor : **TAKAICHI, Akihisa 172-3,**
**Aza-nakajima Takashima**
**Naruto-cho Naruto-shi**
**Tokushima 772 (JP)**
Inventor : **OKAMOTO, Toshihiko 632, Ko**
**Kokufu-cho Tokushima-shi**
**Tokushima 779-31 (JP)**
Inventor : **MATSUMOTO, Toshiaki Imagire-Ryo**
**463-10, Kagasuno Kawauchi-cho**
**Tokushima-shi Tokushima 771-01 (JP)**
Inventor : **NAKAMURA, Junji Heijoukyodanchi**
**22-402**
**9, Ukyo 5-chome Nara-shi**
**Nara 631 (JP)**
Inventor : **NAKAMURA, Toshio 1-115, Aza-nibu**
**Shinkirai Kitajima-cho**
**Itano-gun Tokushima 771-02 (JP)**

(74) Representative : **Hansen, Bernd, Dr.**
**Dipl.-Chem. et al**
**Hoffmann, Eitle & Partner Patent- und**
**Rechtsanwälte, Postfach 81 04 20**
**D-81904 München (DE)**

Jouve, 18, rue Saint-Denis, 75001 PARIS

## Description

TECHNICAL FIELD

This invention relates to an iron containing preparation for NMR imaging which has a form such as foaming tablet, powder or the like.

Since the beginning of 1970, NMR (Nuclear Magnetic Resonance) is widely utilized as a medical diagnosis apparatus, especially as an imaging means capable of providing soft organization imagings having high resolution and differetiation without using detrimental ray.

That is to say, many atoms have a certain property called as spin to which a small magnetic moment is attached.

When the outer magnetic field does not exist, the configuration of the magnetic moment is irregular, but in the presence of a static magnetic field, the nuclear magnetic moment takes precession to approximately the magnetic field direction, so that a net alignment is generated in the magnetic field. NMR imaging method is achieved by using this priciple. According to the NMR imaging method, when short radio frequency pulse is oscillated from a coil surrounding a patient which is set in a static magnetic field, a configuration based on the new magnetic field and precession in phase are generated by this pulse. On the other hand, when oscillation of the pulse is stopped, the above moment returns to the distribution of the alignment and the irregular distribution of the precession phase on the basis of the former static magnetic field. In such a case, a receivable nuclear magnetic resonance is generated at the receiving coil, and by measuring these NMR signals, a proton density map of the objective tissue can be represented. Also, the NMR signal is largely depending on parameters of the spin-lattice relaxation time ($T_1$, i.e. the time specific to return of nuclear magnetic moment to balance the alignment in a static magnetic field) and the spin-spin relaxation time ($T_2$, i.e. the time specific to return of nuclear magnetic moment to irregular precession phase distribution). Therefore, these measurements can be applied to diagnosis of the tissue state of a patient.

In a NMR imaging method, it is known that physical parameter such as temperature, viscosity and hydration or the like of the tissue is effective to increase the NMR signal strength or contrast of NMR image. However, these methods are apparently not suitable in clinical view. A method for enhancing the contrast of NMR image which is known in the present stage is to use a paramagnetic compound, as a contrast agent, which decreases the spin-lattice relaxation time ($T_1$) at low concentration thereof, and decreases the spin-spin relaxation time ($T_2$) at a high concentration thereof. The contrast agent is variously researched, and typical examples of the suggested contrast agents are inorganic paramagnetic salts such as of iron, manganese, chromium; or organic chelate complexes which consist of the paramagnetic metal ion mentioned above and one of various complex forming agents which are mainly aminopolycarbxylic acid such as etylenediaminetetraacetic acid or diethylenetriaminepentaacetic acid. The contrast agent is orally or unorally taken in the form of a solution or a colloidal dispersion liquid.

However, all the known contrast agents which are suggested are insufficient in practical view in a NMR imaging method, e.g. easiness for preparing a pharmaceutical form, stability of the pharmaceutical form, easiness for orally taking, taste, toxicity, or the like and also not always sufficient as a contrast agent, e.g. with respect to accuracy and clearness.

The main object of the invention is to provide an iron containing preparation for NMR imaging, which can easily be prepared in a pharmaceutical form, and has an excellent solubility or dispersability in water so as to rapidly and easily dissolve or disperse in water, thereby being suitable to orally taking.

Another object of the invention is to provide an iron containing preparation for NMR imaging, which is excellent in storage stability.

Another object of the invention is to provide an iron containing preparation for NMR imaging which has an effect capable of accurately and clearly imaging abdominal organs as a contrast agent.

According to this invention, there is provided an iron containing preparation for NMR imaging comprising, as essential ingredients, a) 0.1 to 10 % by weight, as converted to iron, of an iron salt b), 8 to 60 % by weight of one or both selected from sodium carbonate and sodium hydrogencarbonate and c) 10 to 70 % by weight of a neutralizing agent.

The preparation of this invention is mainly used in the form of a tablet, granule, powder or capsule.

The preparation of this invention, especially in the form of a podwer or tablet, has excellent dissolution and dispersion properties in water. Therefore, the iron containing preparation is easily dissolved or dispersed in water by merely putting the preparation into water, with generating carbonic acid gas (carbon dioxide) due to neutralization. Therefore, it can easily be administered orally. Also, carbonic acid gas generated in the body of the patient makes the alimentary canal expand and extend, so that the form of the alimentary canal, the state of lumen thereof and the relation between the alimentary canal and other surrounding internal organs can be easily known.

Furthermore, by taking the preparation of this invention, the extremely significant effect that the signal strength of the lumen of the alimentary canal is enhanced so as to be able to image the alimentary canal wall and to enhance the contrast against the adjacent abdominal organs such as pancreas and the like is achieved.

According to this invention, in order to improve preservation stability, there is provided an iron containing preparation for NMR imaging comprising the above iron salt sodium carbonate and/or sodium hydrogencarbonate, the neutralizing agent and potassium carbonate which is a preservation stabilizing agent.

Examples of the iron salt preferably employed in this invention are ammonium iron(II) citrate, ammonium iron(III) citrate, sodium iron(II) citrate, sodium iron(III) citrate, iron(II) citrate, iron(III) citrate, iron(II) gluconate, iron(II) pyrophosphate, iron(III) pyrophosphate, iron lactate, iron(II) sulfate, iron(III) chloride, iron sesquioxide, sodium iron chlorophyn, iron(II) fumarate, iron threonine, iron(II) orotinate, saccharated iron oxide, iron(III) gluconate or the like. These iron containing compounds are excellent in solubility regarding their dispersive property in water. These iron salts are also used as an active component of the therapeutic agent of iron deficiency anemia, an agent for anemia, a hematinic iron agent or the like in pharmaceutical field, and have high safety.

In the iron salts mentioned above, it is preferred to use trivalent iron salt, and especially it is most preferred to use the trivalent citrate type, in view of high safety and imaging( on contrast ) effects, good taste and it is also easily drinkable.

The iron salt is added in the form of powder, the diameter of which is ordinally not more than 200 $\mu$m. Each iron salt may be used alone or in a mixture of 2 kinds or more than 2 kinds thereof. The amount of iron salt to be added is 0.1 to 10 % by weight, preferably 0.5 to 5 % by weight as converted to iron. Within this amount, the preparation of this invention achieves accurate and clear contrast effects in NMR imaging. This amount corresponds with 10 to 300 mg, preferably 25 to 100 mg per one preparation of the foam preparation of this invention.

Sodium carbonate and/or sodium hydrogencarbonate and a neutralizing agent are added as a foam component together with the above iron salt. The neutralizing agent is an acid compound capable of neutralizing sodium hydrogen carbonate and sodium bicarbonate to generate carbonic acid gas. Such a foam has a function capable of expanding and extending the alimentary canal, and therefore it is very advantageous to know the form of the alimentary canal and the state of its lumen from the NMR picture. An example of such a neutralizing agent is an organic acid such as L-tartaric acid, citric acid, fumaric acid, lactic acid, malic acid or ascorbic acid, and it is especially preferred to use L-tartaric acid and/or citric acid.

The amount of the above foam component to be blended is decided so that the solution obtained by dissolving in water indicates acid, especially 3 to 5.5 of pH, preferably 3.5 to 4.6 of pH, whereby the iron containing compound is rapidly dissolved in water. As a more concrete explanation of the blending amount of each ingredient, sodium carbonate and/or sodium hydrogencarbonate is 8 to 60 % by weight, and the neutralizing agent is 10 to 70 % by weight. In case that the preparaion of this invention is used in the form of powder or the like, when the amount of sodium carbonate and/or sodium hydrogencarbonate is 20 to 60 % by weight, excellent imaging effects are obtained, and when the amount of sodium carbonate and/or sodium hydrogencarbonate is 8 to 45 % by weight, taste is improved so as to be agreable to drink. Practically, it is desirable in view of good taste and easiness of taking together with high imaging effects that sodium carbonate is added at 9 to 50 % by weight, preferably 22 to 26 % by weight, and that sodium hydrogencarbonate is 8 to 50 % by weight, preferably 20 to 45 % by weight.

It is suitable that the neutralizing agent is added in the range of 20 to 50 % by weight, preferably 30 to 40 % by weight, and especially it is preferable to use at the same amount as or more than the equivalent of sodium hydrogencarbonate.

According to this invention, in addition to sodium carbonate and/or sodium hydrogencarbonate and a neutralizing agent added as a foam component, it is preferred that potassium carbonate is added as a preservation stabilizing agent. That is to say, since sodium carbonate or sodium hydrogencarbonate are neutralized in the presence of water by neutralizing agent such as an organic acid to generate carbonic acid gas and promote the degradation and dissolution of the preparation, for preservation the preparation should be kept under dry conditions as much as possible to avoid foaming. There is, however, a possibility of occuring foam during the preservation due to the presence of water remaining in the preparing process or crystal water, even if it is preserved in a sealed container together with the drying agent. If carbonic acid gas is generated during the preservation, the inner pressure of the sealed container is increased, and results in deformation or damage of the container, or prevents the product from foaming.

Foaming during preservation is accelerated under high temperature condition, and further the generated reaction water and carbonic acid gas accelerate the reaction.

It has now been found that potassium carbonate is very effective to prevent foam during the preservation as mentioned above, and even if the drying agent is not used during preservation, foam can be prevented. In view of securing a high stability of the preparation and easily taking it without deterioration taste, it is suitable that potassium carbonate is added in an amount of 0.2 to 13 % by weight, preferably 0.3 to 3 % by weight, more preferably 0.4 to 1 % by weight per one preparation.

Potassium carbonate used in this invention is not particularly limited, and it is especially preferred to use one having no crystal water, such as potassium carbonate anhydride.

To the preparation in this invention, if necessary, various additives ordinally known, such as a vehicle, a binding agent, a disintegrator, a lubricant, a thicknener, a surface active agent, an osmotic pressure adjusting agent, an electrolyte, a sweetening agent, perfume, coloring matter, pH adjusting agent or the like, can be added, in addition to the above iron salt and foam components. Examples of the vehicle are starches such as wheat starch, potato starch, corn starch, dextrin; saccharides such as sucrose, glucose, fructose, maltose, xylulose, and lactose; sugar alcohols such as sorbitol, mannitol, maltitol and, xylitol; saccharide-transglycoside such as coupling sugar or palathinose; calcium phosphate; and calcium sulfate. Examples of the binding agent or thickener are starch, saccharides, gelatin, gum arabic, dextrin, methyl cellulose, CMC-Na, polyvinyl pyrrolidone, polyvinyl alcohol, hydroxypropyl cellulose, xanthan gum, pectin, tragacanth gum, casein, and alginic,. Examples of lubricant are leucine, isoleucine, L-valine, sugar-ester, hardened oil, stearic acid, magnesium stearate, talc and, macrogol. Examples of disintegrator are avicel®, CMC and CMC-Ca. Examples of surface active agent are polysorbate and lecithin. Examples of sweetening agent are saccharides; sugar alcohols; dipeptides such as aspartame, alitame; stevia and saccharin.

The suitable amounts of these additives can be decided in view of the relationship between the additives and the essential ingredients, the properties of preparation, the process for preparing it or the like.

Furthermore, the suitable amount of various vitamins , especially cyanocobalamin or ascorbic acid (vitamin C) may be added to the preparation. Therefore, it is also possible to supply vitamins to the body. The amount of the vitamins to be added is not limted, and vitamin C may be added at the amount of not excessing 30 % by weight, preferably about 5 to 25 % by weight in view of taste.

The preparation of this invention is not only in the form of a tablet, but may also be in the solid form such as granule, powder or capsule.

In preparing the preparation of this invention, usual methods may be employed.

For example, a tablet can be prepared by a method for directly pressurizing powders or a method for dry or wet pressurizing granule, after weighing and mixing the prescribed amount of each ingredient. Also, the powder can be prepared by weighing and mixing the prescribed amount of each ingredient followed by folding. Granule can be prepared by drying to form particles followed by folding, after weighing and mixing the prescribed amount of each ingredient.

The preparation of this invention which is in the form of foam tablet or powder is put into water to dissolve or disperse, and is then taken orally. Conversely, the preparation of this invention may be orally taken in its unchanged form followed by drinking water.

The dose of the preparation of this invention should be suitably decided in compliance with the internal organs, and in general, may be taken by dissolving 1.5 to 6 g of the preparation in 100 to 300 ml of water. In the case of contrast imaging of the pancreas, 1 or 2 tablets which are prepared at about 1.5 to 6 g per one tablet are taken by dissolving in 100 to 300 ml of water.

The preparation of this invention can be utilized in NMR diagnosis of the alimentary canal, i.e. walls of the alimentary canal such as the stomach, duodenum, small intestine, large intestine or the like; or the pancreas, liver, peritoneum, mesenterium or the like. In this case, the preparation of this invention is suitable for contrast imaging representation between the alimentary canal and the parenchyma of internal organs, whereby the $T_1$ value is shortened.

## BRIEF EXPLANATION OF THE DRAWINGS

Fig. 1 is a NMR imaging photography of an abdominal part before taking the preparation of Example 1;

Fig. 2 is a NMR imaging photography of an abdominal part after taking the preparation of Example 1;

Figs. 3 and 4 are NMR imaging photographies of an abdominal part of the other subject after taking the preparation of Example 1;

Fig. 5 is a NMR imaging photography of an abdominal part before taking the preparation of Example 20;

Fig. 6 is a NMR imaging photography of an abdominal part of the other subject after taking the preparation of Example 20;

Figs. 7 to 9 are NMR imaging photographies of an abdominal part of the other subject after taking the preparation of Example 20;

INDUSTRIAL APPLICABILITY

As mentioned above, the preparation of this invention makes it possible to take it orally with ease, and to expand and extend the alimentary canal by foaming of the foaming ingredients. As a result, the form of alimentary canal, the state of its lumen and the relationship between the alimentary canal and the surrounding organs can be easily known. Furthermore, the preparation of this invention has an excellent imaging effect enhancing the signal strength in the alimentary canal. Thus, it is expected to improve the accuracy of diagnosis of various diseases.

Also, by adding potassium carbonate to the foam preparation, foaming and alteration during preservation can be prevented, and as a result, the preparation of this invention is superior in preservation stability.

EXAMPLES

The Examples of this invention are explained below in detail. In each example, "parts" and "%" mean "parts by weight" and "% by weight", respectively, except for special remarks.

Example 1

After mixing each ingredient at the ratio shown belox, the foam tablets (4.3 g per tablet) was pharmaceutically prepared from the mixture by a method for directly pressurizing powder.

| (Ingredients) | ( % ) |
|---|---|
| Granulated sugar | 37 |
| L-Ascorbic acid | 12 |
| L-Tartaric acid | 22 |
| Aspartame | 0.8 |
| Sodium hydrogencarbonate | 23 |
| Ammonium iron citrate | 3.4 |
| (25 mg/4.3 g as converted to iron) | |
| Cyanocobalamin | trace amount |
| perfume and coloring | proper amount |
| Total | 100 |

Example 2 to 8

Foam tablets having compositions shown in Table 1 were prepared by the same method as in Example 1.

Table 1.

| Ingredients | | Example No. | | | | | | |
|---|---|---|---|---|---|---|---|---|
| | | 2 | 3 | 4 | 5 | 6 | 7 | 8 |
| Granulated sugar | (parts) | 34 | 30 | 26 | 14 | 17 | 39 | 28 |
| L-Ascorbic acid | (parts) | 12 | 12 | 12 | 16 | 16 | 12 | 12 |
| L-Tartaric acid | (parts) | 22 | 22 | 22 | 30 | 30 | 23 | 27 |
| Aspartame | (parts) | 0.8 | 0.8 | 0.8 | 1.0 | 1.0 | 0.8 | 0.8 |
| $NaHCO_3$ | (parts) | 23 | 23 | 23 | 31 | 31 | 20 | 25 |
| Ammonium iron citrate | (parts) | 6.8 | 10.2 | 14 | 6.8 | 3.4 | 3.4 | 6.8 |
| Cyanocobalamin | (parts) | * | * | * | * | * | * | * |
| Perfume and coloring | (parts) | ** | ** | ** | ** | ** | ** | ** |
| Preparation weight (g/one tablet) | | 4.3 | 4.3 | 4.3 | 4.3 | 4.3 | 4.3 | 4.3 |
| Iron content/one tablet (mg) | | 50 | 75 | 100 | 50 | 25 | 25 | 50 |

* indicates "a trace amount of cyanocobalamin"

** indicates "a suitable amount of perfume and coloring matter"

EP 0 401 377 B1

Example 9 to 20

The prescribed amount of each ingredient shown in Table 2 was weighed and mixed, and further sweetening agent and perfume were added at suitable amounts. Then, by folding the mixture, foam powders having a weight (mg/one package) shown in the same table were prepared.

EP 0 401 377 B1

Table 2.

| Ingredients | Example No. | | | | | | | | | | | |
|---|---|---|---|---|---|---|---|---|---|---|---|---|
| | 9 | 10 | 11 | 12 | 13 | 14 | 15 | 16 | 17 | 18 | 19 | 20 |
| L-Tartaric acid (mg) | 893 | 893 | 893 | 893 | 893 | 447 | 1786 | 893 | 893 | 447 | 1786 | 1100 |
| $NaHCO_3$ (mg) | 1000 | 1000 | 1000 | 1000 | 1000 | 500 | 2000 | 500 | 2000 | 1000 | 1000 | 1250 |
| Ammonium iron citrate (mg) | 60 | 150 | 300 | 600 | 1200 | 600 | 600 | 600 | 600 | 600 | 600 | 600 |
| Total (mg/one package) | 1953 | 2043 | 2193 | 2493 | 3093 | 1547 | 4386 | 1993 | 3493 | 2047 | 3386 | 2950 |
| Iron content /one package(mg) | 10 | 25 | 50 | 100 | 200 | 100 | 100 | 100 | 100 | 100 | 100 | 100 |

NMR imaging test (I)

1, 1.5, 2 and 2.5 foam tablets (including 25 mg, 37.5 mg, 50 mg and 62.5 mg of iron, respectively) prepared in Example 1 were taken by four healthy and ordinary subjects (No.s 1 to 4) by dissolving in 140 ml of water, respectively. NMR imaging is carried out before and after taking the foam tablets. In such a case, photographs of $T_1$ enhancement image (SE 500 to 600/17 m sec.) and $T_2$ enhancement image (SE 2000/23.90 m sec.) were taken. $T_1$ and $T_2$ values were measured from images of SE 500/23 and 2000/23.90 by double point method. Also, as a measurement equipment, 1.5T MRI (Magnetom) manufactured by Siemens, W. Germany, and 8 to 10 mm of slice thickness and 4 to 5 mm of slice interval were set.

$T_1$ and $T_2$ values in the stomach which were obtained by the above test are shown in Table 3.

Table 3

| Subject No. | Dose (mg of iron) | Before taking (Stomach) | After taking (Stomach) |
|---|---|---|---|
| | | $T_1$ / $T_2$ | $T_1$ / $T_2$ |
| 1 | 25.0 | 3111 / 122 | 2213 / 149 |
| 2 | 37.5 | 3635 / 193 | 744 / 179 |
| 3 | 50.0 | 2379 / 178 | 573 / 272 |
| 4 | 62.5 | 3305 / 202 | 565 / 307 |

The following matter becomes apparent from Table 3. Enhancement of the liquid contained in the stomach is recognized in all of the four doses. Especially, when the dose is 25 mg and 62.5 mg of iron, enhancement of the liquid contained in the stomach is remarkable, and images of the stomach wall and pancreas, especially of the head of the pancreas become clear. As to the degree of enhancement, when the dose is 50 mg of iron, the signal strength of the above liquid contained in the stomach is slightly less than that of fatty tissue in the abdominal cavity, and therefore the above liquid can be distinguished from the above fat.

It was also recognized that the foam tablets obtained in Examples 2 to 11 show the same enhancement as that of each subject number at the same dose of iron as the above test. Accordingly, the foam tablets obtained in each Example can be sufficiently applied to abdominal diagnosis using NMR.

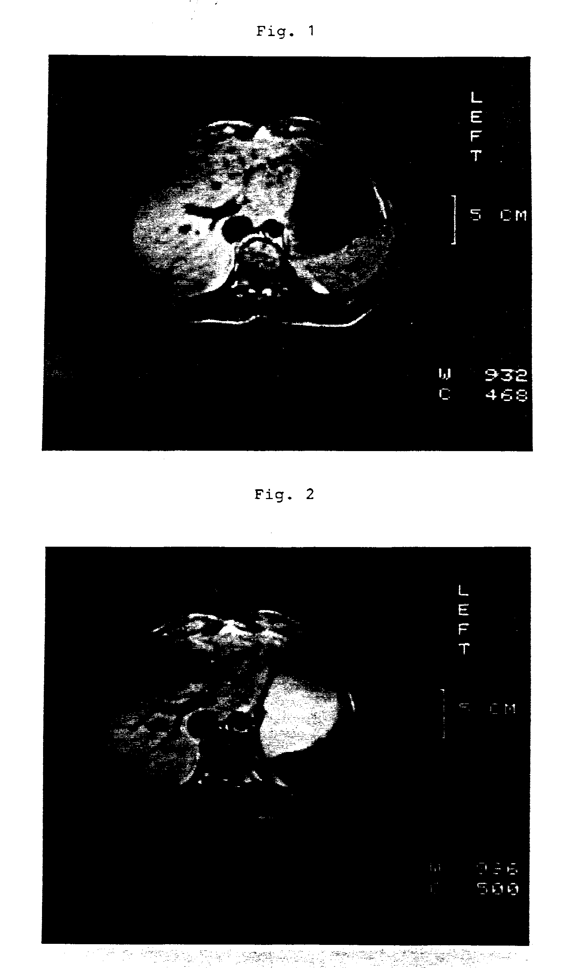

These test results were confirmed by practically administrating the foam tablets obtained in each Example and taking photographs of the abdominal image. That is, as shown in Fig. 1 which is the $T_1$ enhancement image of the abdominal part of subject No. 4 before taking, since the inner part of stomach is filled with water and the signal is weak, the inner part of the stomach is represented by grey or black color, and it is hard to distinguish the alimentary canal from other adjacent organs. On the other hand, as shown in Fig. 2 which is the $T_1$ enhancement image after taking, time $T_1$ in the stomach is shortened, the signal strength is increased, and therefore the distinction between the alimentary canal and other adjacent organs is clear.

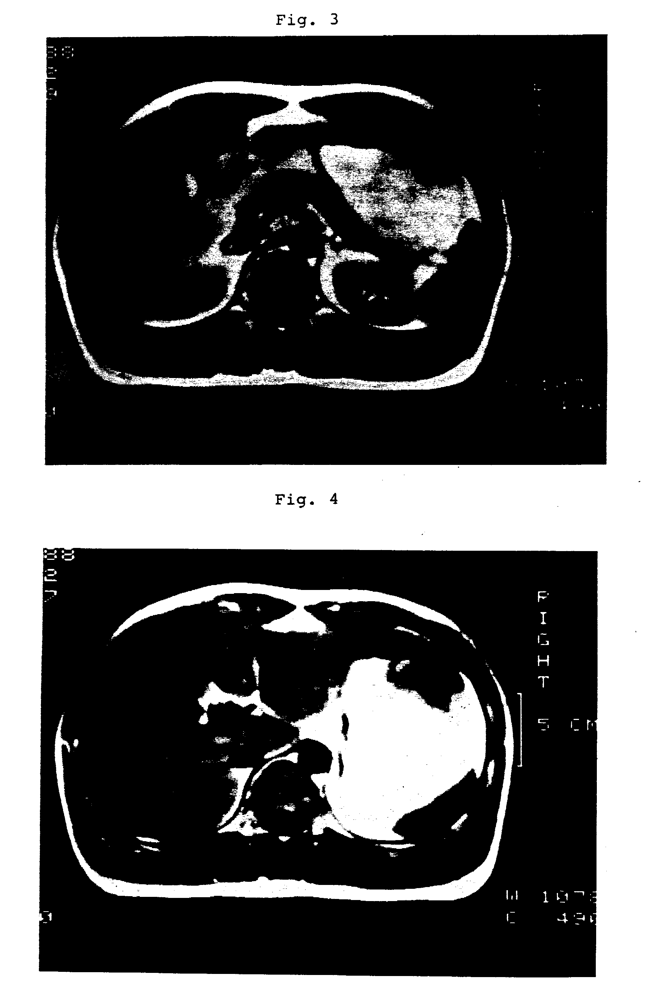

Also, as shown in Figs. 3 and 4, according to $T_1$ enhancement images after taking, the distinction between the alimentary canal and other adjacent organs is clear. Especially, from Fig. 3, the border between the pancreas and other internal organs can be clearly confirmed; the head of the pancreas which is difficult to detect anatomically is apparently recognized; other organs such as spleen tail of pancreas, body of pancreas, liver, kidneys, blood vessel or the like were also recognized clearly; and further the stomach wall was clearly identified.

NMR imaging test (II)

One package of the foam powder (including 100 mg of iron) prepared in Example 20 was taken by a healthy and ordinary subject by dissolving in 140 ml of water, and further 150 ml of water were given to the subject.

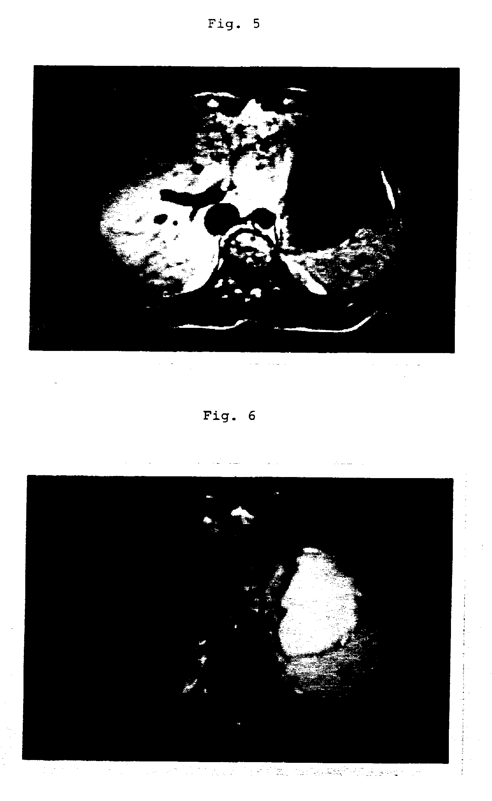

Figs. 5 and 6 are photographs for imaging the abdominal part of the subject before and after taking the foam powder. Fig. 5 is a $T_1$ enhancement image of the stomach part in the condition that water was given to expand the alimentary canal. As shown in Fig. 5, the signal of water is weak, whereby the inner part of the stomach is represented by grey or black color, and the distinction between the wall and lumen of the alimentary canal is unclear. Furthermore, it is difficult to recognize a distinction between the alimentary canal and the adjacent organs such as pancreas, liver, spleen,peritoneum or the like.

On the other hand, the signal strength in the stomach after taking is increased as shown in the $T_1$ enhancement image of Fig. 6, the inner part of the stomach is drawn out by white color, and is contrasted to the surrounding organs. Also, as mentioned later, the stomach wall and the duodenum wall are well recognized, and the tail and head of the pancreas are clearly distinguished from the surrounding organs and the alimentary canal.

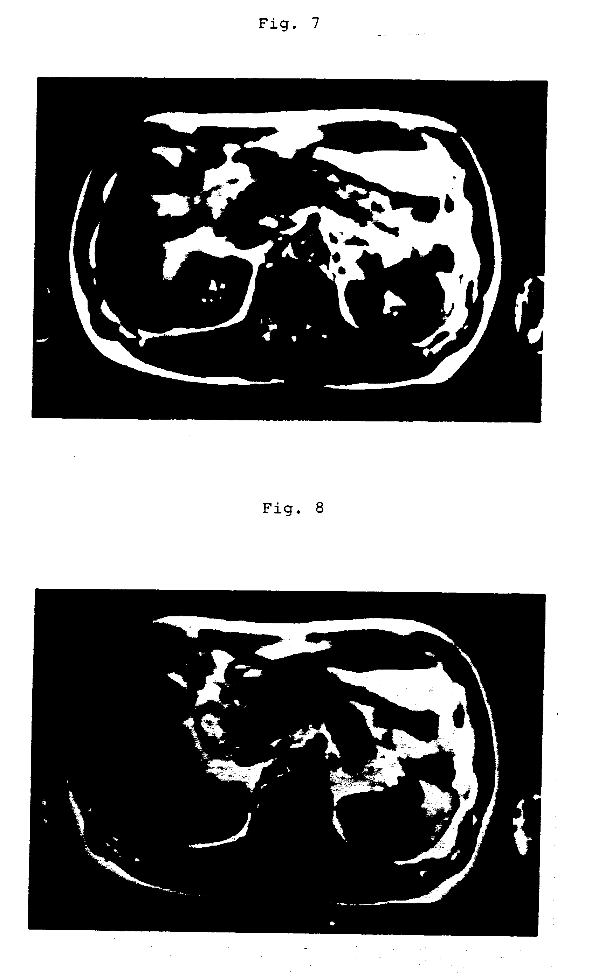

Fig. 7 is the $T_1$ enhancement image after taking one package of foam powder obtained by Example 20 with 300 ml of water. In general, it is difficult to take an image of the head of pancreas, since its $T_1$ signal approximates to that of the duodenum. However, by taking the foam powder of this Example, since the duodenum is expanded and extended by generating carbonic acid gras, and the signal strength is increased, the head of the pancreas can be very clearly drawn out. Similarly, the stomach part is fully expanded and extended by water and carbonic acid gas, the border between the stomach and the body of the pancreas is distinct, and contrast is enhanced.

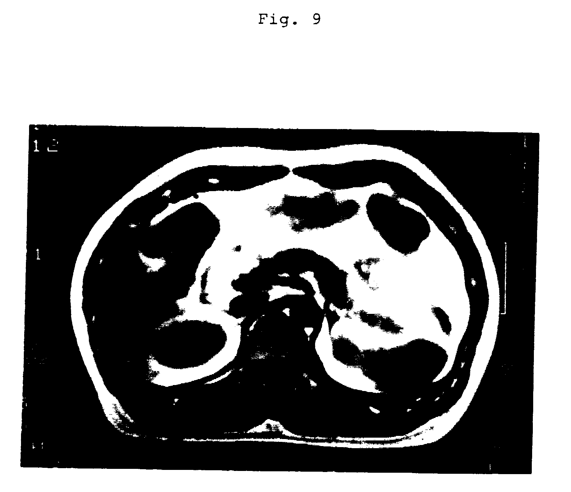

It is understood from Fig. 8 that the distinction between the wall of the duodenum and the inner wall is clear, since the duodenum is expanded and extended by generating carbonic acid gas. It is also understood from Fig. 9 that the duodenum is expanded and extended for the same reason as in Fig. 8.

Accordingly, from the results shown in Figs. 5 to 9, the shape of the abdominal organs and the relationship between the same and other organs can be accurately and clearly known by taking the foam powder of this Example, whereby it is expected to improve the accuracy of diagnosis against various diseases.

Example 21 (including potassium carbonate)

Foam tablet having the composition shown below was prepared in the same manner as in Example 1.

| (Ingredients) | (%) |
|---|---|
| Granulated sugar | 40 |
| L-Tartaric acid | 29 |
| Aspartame | 0.8 |
| Sodium hydrogencarbonate | 21 |
| Ammonium iron citrate | 3.6 |
| Potassium carbonate | 0.5 |
| Cyanocobalamin | trace amount |
| Sweetening agent | proper amount |
| Perfume and coloring | proper amount |
| Total | 100    (4.0 g) |

Stability test

The Foam tablet obtained in Example 21 was stored in a constant temperature room kept at 37 °C, together with the comparative foam tablet which was prepared by the same manner as in Example 21 except for not adding potassium carbonate, and swelling test of the wrapping sheet, discoloration test of tablets, solubility in water and change of taste were examined with time. As a result, it is understood that the foam tablet of Example 21 with added potassium carbonate had low swell, discoloration, dissolving time and change of taste in comparison with the comparative foam tablet, and therefore is superior to the compartive foam tablet in preservation stability.

## Claims

1. Iron containing preparation for NMR imaging which comprises, as necessary ingredients,
   a.) 0.1 to 10 % by weight, as converted in iron, of an iron salt;
   b.) 8 to 60 % by weight of sodium carbonate and/or sodium hydrogencarbonate; and
   c.) 10 to 70 % by weight of a neutralizing agent for b.)

2. Iron containing preparation for NMR imaging according to claim 1, wherein said iron salt is at least one selected from ammonium iron(II) citrate, ammonium iron(III) citrate, sodium iron(II) citrate, sodium iron(III) citrate, iron(II) citrate, iron(III) citrate, iron(II) gluconate, iron(II) pyrophosphate, iron(III) pyrophosphate, iron lactate, iron(II) sulfate, iron(III) chloride, iron sesquioxide, sodium iron chlorophyn, iron(II) fumarate, iron threonine, iron(II) orotinate, saccharated iron oxide, and iron(III) gluconate.

3. Iron containing preparation for NMR imaging according to claim 2, wherein said iron salt is a trivalent iron salt.

4. Iron containing preparation for NMR imaging according to claim 3, wherein said iron salt is a trivalent iron salt of citrate type.

5. Iron containing preparation for NMR imaging according to claim 1, wherein said iron salt is added in an amount of 0.5 to 5 % by weight as converted in iron.

6. Iron containing preparation for NMR imaging according to claim 1, wherein said neutralizing agent is selected from L-tartaric acid, citric acid, fumaric acid, lactic acid, malic acid and ascorbic acid.

7. Iron containing preparation for NMR imaging according to claim 6, wherein said neutralizing agent is L-tartaric acid or citric acid.

8. Iron containing preparation for NMR imaging according to claim 1, wherein the pH of a solution which is obtained by dissolving said preparation in water is 3 to 5.5.

9. Iron containing preparation for NMR imaging according to claim 8, wherein the pH of said solution is 3.5 to 4.6.

10. Iron containing preparation for NMR imaging according to claim 1, wherein the sodium carbonate and/or sodium hydrogencarbonate is added in an amount of 20 to 60 % by weight.

11. Iron containing preparation for NMR imaging according to claim 10, wherein the sodium carbonate and/or sodium hydrogencarbonate is added in an amount of 8 to 45 % by weight.

12. Iron containing preparation for NMR imaging according to claim 1, wherein sodium carbonate is added in an amount of 9 to 50 % by weight.

13. Iron containing preparation for NMR imaging according to claim 12, wherein sodium carbonate is added in an amount of 22 to 26 % by weight.

14. Iron containing preparation for NMR imaging according to claim 1, wherein sodium hydrogencarbonate is added in an amount of 8 to 50 % by weight.

15. Iron containing preparation for NMR imaging according to claim 14, wherein sodium hydrogencarbonate is added in an amount of 20 to 45 % by weight.

16. Iron containing preparation for NMR imaging according to claim 1, wherein said neutralizing agent is added in an amount of 20 to 50 % by weight.

17. Iron containing preparation for NMR imaging according to claim 16, wherein said neutralizing agent is in an amount of 30 to 40 % by weight.

18. Iron containing preparation for NMR imaging according to claim 1, wherein the preparation form is one dissolved or dispersed in water.

19. Iron containing preparation for NMR imaging according to claim 18, wherein the preparation form is a foam powder.

20. Iron containing preparation for NMR imaging according to claim 18, wherein the preparation form is a foam tablet.

21. Iron containing preparation for NMR imaging according to claim 1 which comprises, as necessary ingredients, an iron salt, sodium carbonate and/or sodium hydrogencarbonate, a neutralizing agent and potassium carbonate as a preservation stabilizing agent.

22. Iron containing preparation for NMR imaging according to claim 21, wherein potassium carbonate is added in an amount of 2 to 13 % by weight.

23. Iron containing preparation for NMR imaging according to claim 22, wherein potassium carbonate is added in an amount of 0.3 to 3 % by weight.

24. Iron containing preparation for NMR imaging according to claim 23, wherein potassium carbonate is added in an amount of 0.4 to 1 % by weight.

25. Use of an iron containing preparation of claim 1 for preparing a composition to be used in an NMR imaging method.


**Patentansprüche**

1. Eisen enthaltende Präparation zur NMR-Bildwiedergabe, die als notwendige Bestandteile umfaßt
    a) 0,1 bis 10 Gewichts-%, umgerechnet auf Eisen, eines Eisensalzes;
    b) 8 bis 60 Gewichts-% Natriumcarbonat und/oder Natriumhydrogencarbonat; und
    c) 10 bis 70 Gewichts-% eines für b) neutralisierenden Mittels.

2. Eisen enthaltende Präparation zur NMR-Bildwiedergabe nach Anspruch 1, worin das Eisensalz wenigstens eines ist, ausgewählt aus Ammonium-Eisen(II)-citrat, Ammonium-Eisen(III)-citrat, Natrium-Eisen(II)-citrat, Natrium-Eisen(III)-citrat, Eisen(II)-citrat, Eisen(III)-citrat, Eisen(II)-gluconat, Eisen(II)-pyrophosphat, Eisen(III)-pyrophosphat, Eisenlactat, Eisen(II)-sulfat, Eisen(III)-chlorid, Eisensesquioxid, Natrium-Eisenchlorophyn, Eisen(II)-fumarat, Eisenthreonin, Eisen(II)-orotinat, Saccharid-Eisenoxid und Eisen(III)-gluconat.

3. Eisen enthaltende Präparation zur NMR-Bildwiedergabe nach Anspruch 2, worin das Eisensalz ein dreiwertiges Eisensalz ist.

4. Eisen enthaltende Präparation zur NMR-Bildwiedergabe nach Anspruch 3, worin das Eisensalz ein dreiwertiges Eisensalz vom Citrat-Typ ist.

5. Eisen enthaltende Präparation zur NMR-Bildwiedergabe nach Anspruch 1, worin das Eisensalz in einer Menge von 0,5 bis 5 Gewichts-%, umgerechnet auf Eisen, hinzugesetzt ist.

6. Eisen enthaltende Präparation zur NMR-Bildwiedergabe nach Anspruch 1, worin das neutralisierende Mittel ausgewählt ist unter L-Weinsäure, Citronensäure, Fumarsäure, Milchsäure, Äpfelsäure und Ascorbinsäure.

7. Eisen enthaltende Präparation zur NMR-Bildwiedergabe nach Anspruch 6, worin das neutralisierende Mittel L-Weinsäure oder Citronensäure ist.

8. Eisen enthaltende Präparation zur NMR-Bildwiedergabe nach Anspruch 1, worin der pH-Wert einer Lö-

EP 0 401 377 B1

sung, die durch Auflösen der genannten Präparation in Wasser erhalten wird, 3 bis 5,5 beträgt.

9. Eisen enthaltende Präparation zur NMR-Bildwiedergabe nach Anspruch 8, worin der pH der genannten Lösung 3,5 bis 4,6 beträgt.

10. Eisen enthaltende Präparation zur NMR-Bildwiedergabe nach Anspruch 1, worin das Natriumcarbonat und/oder Natriumhydrogencarbonat in einer Menge von 20 bis 60 Gewichts-% hinzugesetzt ist.

11. Eisen enthaltende Präparation zur NMR-Bildwiedergabe nach Anspruch 10, worin das Natriumcarbonat und/oder Natriumhydrogencarbonat in einer Menge von 8 bis 45 Gewichts-% hinzugesetzt ist.

12. Eisen enthaltende Präparation zur NMR-Bildwiedergabe nach Anspruch 1, worin Natriumcarbonat in einer Menge von 9 bis 50 Gewichts-% hinzugesetzt ist.

13. Eisen enthaltende Präparation zur NMR-Bildwiedergabe nach Anspruch 12, worin Natriumcarbonat in einer Menge von 22 bis 26 Gewichts-% hinzugesetzt ist.

14. Eisen enthaltende Präparation zur NMR-Bildwiedergabe nach Anspruch 1, worin Natriumhydrogencarbonat in einer Menge von 8 bis 50 Gewichts-% hinzugesetzt ist.

15. Eisen enthaltende Präparation zur NMR-Bildwiedergabe nach Anspruch 14, worin Natriumhydrogencarbonat in einer Menge von 20 bis 45 Gewichts-% hinzugesetzt ist.

16. Eisen enthaltende Präparation zur NMR-Bildwiedergabe nach Anspruch 1, worin das neutralisierende Mittel in einer Menge von 20 bis 50 Gewichts-% hinzugesetzt ist.

17. Eisen enthaltende Präparation zur NMR-Bildwiedergabe nach Anspruch 16, worin das neutralisierende Mittel in einer Menge von 30 bis 40 Gewichts-% hinzugesetzt ist.

18. Eisen enthaltende Präparation zur NMR-Bildwiedergabe nach Anspruch 1, worin die Präparationsform eine in Wasser gelöste oder dispergierte ist.

19. Eisen enthaltende Präparation zur NMR-Bildwiedergabe nach Anspruch 18, worin die Präparationsform ein Aufschäum-Pulver ist.

20. Eisen enthaltende Präparation zur NMR-Bildwiedergabe nach Anspruch 18, worin die Präparationsform eine Aufschäum-Tablette ist.

21. Eisen enthaltende Präparation zur NMR-Bildwiedergabe nach Anspruch 1, die als notwendige Bestandteile umfaßt ein Eisensalz, Natriumcarbonat und/oder Natriumhydrogencarbonat, ein neutralisierendes Mittel und Kaliumcarbonat als ein die Erhaltung stabilisierendes Mittel.

22. Eisen enthaltende Präparation zur NMR-Bildwiedergabe nach Anspruch 21, worin Kaliumcarbonat in einer Menge von 0,2 bis 13 Gewichts-% hinzugesetzt ist.

23. Eisen enthaltende Präparation zur NMR-Bildwiedergabe nach Anspruch 22, worin Kaliumcarbonat in einer Menge von 0,3 bis 3 Gewichts-% hinzugesetzt ist.

24. Eisen enthaltende Präparation zur NMR-Bildwiedergabe nach Anspruch 23, worin Kaliumcarbonat in einer Menge von 0,4 bis 1 Gewichts-% hinzugesetzt ist.

25. Verwendung einer Eisen enthaltenden Präparation nach Anspruch 1 zur Herstellung einer Zusammensetzung, die in einer NMR-Bildwiedergabemethode verwendet wird.

**Revendications**

1. Préparation contenant du fer pour imagerie RMN qui comprend, comme ingrédients nécessaires:
   a) 0,1 à 10 % en poids, sous forme convertie en fer, d'un sel de fer;
   b) 8 à 60 % en poids de carbonate de sodium et/ou de carbonate acide de sodium, et

13

c) 10 à 70 % en poids d'un agent neutralisant pour b).

2. Préparation contenant du fer pour imagerie RMN selon la revendication 1, dans laquelle ledit sel de fer est au moins un sel choisi parmi : citrate de fer (II) et d'ammonium, citrate de fer (III) et d'ammonium, citrate de fer (II) et de sodium, citrate de fer (III) et de sodium, citrate de fer (II), citrate de fer (III), gluconate de fer (II), pyrophosphate de fer (II), pyrophosphate de fer (III), lactate de fer, sulfate de fer (II), chlorure de fer (III), sesquioxyde de fer, chlorophyne de fer et de sodium, fumarate de fer (II), thréonine de fer, orotinate de fer (II), oxyde de fer saccharisé, et gluconate de fer (III), etc.

3. Préparation contenant du fer pour imagerie RMN selon la revendication 2, dans laquelle ledit sel de fer est un sel de fer trivalent.

4. Préparation contenant du fer pour imagerie RMN selon la revendication 3, dans laquelle ledit sel de fer est un sel de fer trivalent du type citrate.

5. Préparation contenant du fer pour imagerie RMN selon la revendication 1, dans laquelle ledit sel de fer est ajouté en une quantité de 0,5 à 5 % en poids, convertie en fer.

6. Préparation contenant du fer pour imagerie RMN selon la revendication 1, dans laquelle ledit agent neutralisant est choisi parmi l'acide L-tartrique, l'acide citrique, l'acide fumarique, l'acide lactique, l'acide malique et l'acide ascorbique.

7. Préparation contenant du fer pour imagerie RMN selon la revendication 6, dans laquelle ledit agent neutralisant est l'acide L-tartrique ou l'acide citrique.

8. Préparation contenant du fer pour imagerie RMN selon la revendication 1, dans laquelle le pH d'une solution qui est obtenue en dissolvant ladite préparation dans l'eau est de 3 à 5,5.

9. Préparation contenant du fer pour imagerie RMN selon la revendication 8, dans laquelle le pH de ladite solution est de 3,5 à 4,6.

10. Préparation contenant du fer pour imagerie RMN selon la revendication 1, dans laquelle le carbonate de sodium et/ou le carbonate acide de sodium est ajouté en une quantité de 20 à 60 % en poids.

11. Préparation contenant du fer pour imagerie RMN selon la revendication 10, dans laquelle le carbonate de sodium et/ou le carbonate acide de sodium est ajouté en une quantité de 8 à 45 % en poids.

12. Préparation contenant du fer pour imagerie RMN selon la revendication 1, dans laquelle le carbonate de sodium est ajouté en une quantité de 9 à 50 % en poids.

13. Préparation contenant du fer pour imagerie RMN selon la revendication 12, dans laquelle le carbonate de sodium est ajouté en une quantité de 22 à 26 % en poids.

14. Préparation contenant du fer pour imagerie RMN selon la revendication 1, dans laquelle le carbonate acide de sodium est ajouté en une quantité de 8 à 50 % en poids.

15. Préparation contenant du fer pour imagerie RMN selon la revendication 14, dans laquelle le carbonate acide de sodium est ajouté en une quantité de 20 à 45 % en poids.

16. Préparation contenant du fer pour imagerie RMN selon la revendication 1, dans laquelle ledit agent neutralisant est ajouté en une quantité de 20 à 50 % en poids.

17. Préparation contenant du fer pour imagerie RMN selon la revendication 16, dans laquelle ledit agent neutralisant est ajouté en une quantité de 30 à 40 % en poids.

18. Préparation contenant du fer pour imagerie RMN selon la revendication 1, dans laquelle la forme de préparation est dissoute ou dispersée dans l'eau.

19. Préparation contenant du fer pour imagerie RMN selon la revendication 18, dans laquelle la forme de la préparation est une poudre moussante.

**20.** Préparation contenant du fer pour imagerie RMN selon la revendication 18, dans laquelle la forme de la préparation est un comprimé moussant.

**21.** Préparation contenant du fer pour imagerie RMN selon la revendication 1 qui comprend, comme ingrédients nécessaires, un sel de fer, du carbonate de sodium et/ou du carbonate acide de sodium, un agent neutralisant et du carbonate de potassium comme agent stabilisant la conservation.

**22.** Préparation contenant du fer pour imagerie RMN selon la revendication 21, dans laquelle le carbonate de potassium est ajouté en une quantité de 0,2 à 13 % en poids.

**23.** Préparation contenant du fer pour imagerie RMN selon la revendication 22, dans laquelle le carbonate de potassium est ajouté en une quantité de 0,3 à 3 % en poids.

**24.** Préparation contenant du fer pour imagerie RMN selon la revendication 23, dans laquelle le carbonate de potassium est ajouté en une quantité de 0,4 à 1 % en poids.

**25.** Utilisation d'une préparation contenant du fer selon la revendication 1, pour préparer une composition destinée à être utilisée dans un procédé d'imagerie RMN.

Fig. 1

Fig. 2

Fig. 3

Fig. 4

Fig. 5

Fig. 6

Fig. 7

Fig. 8

Fig. 9